(19) Europäisches Patentamt
European Patent Office
Office européen des brevets

(11) Veröffentlichungsnummer: **0 040 583**
**B1**

(12) # EUROPÄISCHE PATENTSCHRIFT

(45) Veröffentlichungstag der Patentschrift: **13.01.88**

(21) Anmeldenummer: **81810186.7**

(22) Anmeldetag: **15.05.81**

(51) Int. Cl.[4]: **C 07 C 109/12,** C 07 C 121/82, G 03 C 1/84, C 08 K 5/30, G 03 C 11/10

(54) **Verfahren zur Herstellung einer UV-Strahlung absorbierenden Maske.**

(30) Priorität: **21.05.80 CH 3962/80**

(43) Veröffentlichungstag der Anmeldung: **25.11.81 Patentblatt 81/47**

(45) Bekanntmachung des Hinweises auf die Patenterteilung: **13.01.88 Patentblatt 88/2**

(84) Benannte Vertragsstaaten: **BE CH DE FR GB IT LI**

(56) Entgegenhaltungen: **DE-A-1 568 725**

(73) Patentinhaber: **CIBA- GEIGY AG, Klybeckstrasse 141, CH- 4002 Basel (CH)**

(72) Erfinder: **Müller, Beat, Dr., Chemin des Cassettes 7, CH- 1723 Marly (CH)**
Erfinder: **Roth, Martin, Dr., Chemin des Epinettes 12, CH- 1723 Marly (CH)**

(74) Vertreter: **Zumstein, Fritz jun., Dr., Dr. F. Zumstein sen. Dr. E. Assmann Dr. R. Koenigsberger Dipl.- Ing. F. Klingseisen Dr. F. Zumstein jun. Bräuhausstrasse 4, D-8000 München 2 (DE)**

**EP 0 040 583 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein Verfahren zur Herstellung einer UV-Strahlung absorbierenden Maske.

Aus der DE-A-1 568 725 sind Hydrazonderivate bekannt, die zwei versalzte Säuregruppen enthalten und als UV-Absorber verwendet werden können. Wegen der beiden anionischen Gruppen können diese Verbindungen jedoch nicht an saure Bindemittel fixiert werden.

Gegenstand der vorliegenden Erfindung ist ein Verfahren zur Herstellung einer UV-Strahlung absorbierenden Maske, bei dem man ein saures photopolymerisierbares Polymer bildmässig belichtet, anschliessend entwickelt und an den belichteten Stellen gleichzeitig oder nach der Entwicklung mit einem kationischen Hydrazonderivat der folgenden Formel (1) behandelt,

$$(1) \quad \left[ A_1-\underset{\underset{\displaystyle B_1}{|}}{N}-N=CH-(CH=CH)_m-Z_1-\overset{\overset{\displaystyle R_1}{|}}{\underset{\underset{\displaystyle R_3}{|}}{\overset{\oplus}{N}}}-R_2 \right] \quad X^{\ominus} ,$$

worin

$A_1$ ein gegebenenfalls mit Alkyl oder Alkoxy mit je 1 bis 4 Kohlenstoffatomen, Hydroxyl oder Halogen substituierter aromatischer Rest,

$B_1$ Wasserstoff, gegebenenfalls mit Methoxy, Hydroxyl, Cyano, Halogen, Carbalkoxy mit 2 bis 9 Kohlenstoffatomen oder Carbonamido substituiertes Alky mit 1 bis 8 Kohlenstoffatomen, gegebenenfalls mit Alkyl oder Alkoxy mit je 1 oder 2 Kohlenstoffatomen substituiertes Cycloalkyl mit 5 oder 6 Ringkohlenstoffatomen, gegebenenfalls im Arylteil mit Alkyl oder Alkoxy mit je 1 bis 4 Kohlenstoffatomen substituiertes Aralkyl mit 1 bis 4 Kohlenstoffatomen im Alkylteil, Naphthyl oder gegebenenfalls mit Alkoxy mit 1 bis 4 C-Atomen substituiertes Phenyl ist,

$R_1$, $R_2$ und $R_3$ unabhängig voneinander gegebenenfalls mit Methoxy, Hydroxy, Cyano, Halogen oder Carbonamido substituiertes Alkyl mit je 1 bis 8 Kohlenstoffatomen, gegebenenfalls mit Methyl oder Methoxy substituiertes Cycloalkyl mit 5 oder 6 Ringkohlenstoffatomen, gegebenenfalls im Phenylteil mit Alkyl oder Alkoxy mit 1 bis 4 C-Atomen substituiertes Phenylalkyl mit 1 bis 4 Kohlenstoffatomen im Alkylteil oder Polyoxyalkylem sind,

$Z_1$ gegebenenfalls mit Hydroxyl, Cyano, Nitro, Halogen, Alkyl oder Alkoxy mit je 1 bis 4 Kohlemstoffatomen substituiertes Arylen,

$X^{\ominus}$ ein Anion einer gegebenenfalls mehrbasischen Mineralsäure,

und

m 0, 1, 2 oder 3 sind.

Der Substituent $A_1$ bedeutet einen aromatischen Rest, wie z. B. Phenyl oder Naphthyl. Vorzugsweise kommt Phenyl im Frage. Substituenten für $A^1$ können Alkyl oder Alkoxy mit je 1 bis 4, insbesondere 1 oder 2 Kohlenstoffatomem, Hydroxyl oder Halogen sein. Bevorzugte Halogene sind Chlor und Brom. Chlor ist besonders geeignet. Der aromatische Rest kann 1 oder 2 Substituenten enthalten.

Wenn der Substituent $B_1$ Alkyl mit 1 bis 8 Kohlenstoffatomen bedeutet, können die Alkylreste geradkettig oder verzweigt sein. Beispielsweise kommen in Frage: Methyl, Äthyl, Propyl, i-Propyl, Butyl, i-Butyl, tert.-Butyl, Amyl, tert.-Amyl, 1,1,3,3-Tetramethylbutyl, 1-Methyläthylpentyl, Hexyl, 1-Methylpentyl, Neopentyl, 1- oder 2-Methylhexyl, Heptyl, n-Octyl, tert. Octyl oder 2-Äthylhexyl. Besonders geeignet sind Alkylreste mit 1 bis 4, insbesondere 1 oder 2 Kohlenstoffatomen. Als Substituenten der Alkylreste kommen Methoxy, Hydroxyl Cyano, Halogen, Carbalkoxy mit 2 bis 9 insbesondere 2 bis 5 Kohlenstoffatomen oder Carbonamido in Betracht. Bevorzugt sind Hydroxyl, Cyano und Carbomethoxy. Bevorzugte Halogene sind Fluor, Chlor und Brom wovon Chlor besonders infrage kommt.

Bedeutet $B_1$ Cycloalkyl, so bestehen die Ringsysteme aus 5 vorzugsweise 6 Kohlenstoffatomen. Sie können mit Alkyl oder Alkoxy mit je 1 oder 2 Kohlenstoffatomen, insbesondere Methyl oder Methoxy substituiert sein.

In der Bedeutung von Aralkyl besitzt $B_1$ 1 bis 4, insbesondere 1 oder 2 Kohlenstoffatome im Alkylteil. Als Arylteil kommen Phenyl oder Naphthyl infrage. Phenyl ist besonders geeignet. Der Arylteil kann mit Alkyl oder Alkoxy mit je 1 bis 4, insbesondere 1 oder 2 Kohlenstoffatomen substituiert sein.

Bei $B_1$ in der Bedeutung von Phenyl oder Naphthyl ist Phenyl bevorzugt. Der Phenylring kann mit Alkoxy mit 1 bis 4, insbesondere 1 oder 2 Kohlenstoffatomen substituiert sein.

Wenn die Reste $R_1$, $R_2$ und $R_3$ unabhängig voneinander Alkyl mit je 1 bis 8 Kohlenstoffatomen bedeuten, kommen dabei die für $B_1$ genannten Alkylreste in Frage. Bevorzugt werden Alkylreste mit 1 bis 4, insbesondere 1 oder 2 Kohlenstoffatomen. Sie können mit Methoxy, Hydroxyl, Cyano, Halogen oder Carbonamido substituiert sein. Der Hydroxylrest ist ganz besonders bevorzugt. Geeignete Halogene sind Fluor, Chlor und Brom. Ein besonders wertvolles Halogen ist Chlor.

Bedeuten $R_1$, $R_2$ und $R_3$ je Cycloalkyl, so werden Ringe mit 5 oder 6 Kohlenstoffatomen bevorzugt. Bevorzugtes Ringsystem ist Cyclohexyl. Als Substituenten kommen dafür Methyl und Methoxy im Frage.

In der Bedeutung von Phenylalkyl enthalten $R_1$, $R_2$ und $R_3$ je 1 bis 4, insbesondere 1 oder 2 Kohlenstoffatome im Alkylteil. Der Phenylteil kann mit Alkyl oder Alkoxy mit je 1 bis 4, insbesondere 1 oder 2 Kohlenstoffatomen weiter substituiert sein. Besonders geeignet ist Benzyl.

Bedeuten $R_1$, $R_2$ und $R_3$ je Polyoxalkylen, so ist $H-(-OH_2H_4-)-_n$ worin n 1, 2 oder 3 ist, bevorzugt. Besonders geeignet ist $H-(-OC_2H_4-)-_n$ mit n in der Bedeutung von 1 oder 2.

$Z_1$ bedeutet Arylen, wie z. B. Phenylen oder Naphthylen, wobei Phenylen besonders geeignet ist. Dieses kann mit Hydroxyl, Cyano, Nitro, Halogen, Alkyl oder Alkoxy mit je 1 bis 4 Kohlenstoffatomen substituiert sein. Methoxy, Hydroxyl und Chlor sind bevorzugte Substituenten. Der Phenylring kann 1 oder 2 der substituenten tragen.

$X^{\ominus}$ bedeutet ein Anion einer gegebenenfalls mehrwertigen Mineralsäure wie z. B. sulfat, Chlorid, Bromid, Jodid oder Methylsulfat und

m ist 0, 1, 2 oder 3, vorzugsweise 0 oder 1.

Besonders geeignete Verbindungen sind solche der Formel (1) worin

$A_1$ als aromatischer Rest Phenyl darstellt.

Bevorzugt sind ferner solche Verbindungen der Formel (1), worin

$A_1$ gegebenenfalls mit Alkyl oder Alkoxy mit je 1 bis 4 Kohlenstoffatomen, Hydroxyl oder Halogen substituiertes Phenyl, wobei der Phenylring 1 oder 2 der substituenten tragen kann,

$B_1$ Wasserstoff gegebenenfalls mit Methoxy, Hydroxyl, Cyano, Chlor, Carbalkoxy mit 2 bis 9 Kohlenstoffatomen oder Carbonamido substituiertes Alkyl mit 1 bis 4 Kohlenstoffatomen, gegebenenfalls mit Methyl oder Methoxy substituiertes Cyclohexyl, gegebenenfalls mit Alkyl oder Alkoxy mit je 1 oder 2 Kohlenstoffatomen im Arylteil substituiertes Aralkyl mit 1 bis 4 Kohlenstoffatomen im Alkylteil oder gegebenenfalls mit Alkoxy mit 1 oder 2 Kohlenstoffatomen substituiertes Phenyl ist,

$R_1$, $R_2$ und $R_3$ unabhängig voneinander gegebenenfalls mit Methoxy, Hydroxyl, Cyano, Chlor oder Carbonamido substituiertes Alkyl mit je 1 bis 4 Kohlenstoffatomen, gegebenenfalls mit Methyl oder Methoxy substituiertes Cyclohexyl, gegebenenfalls im Phenylteil mit Alkyl oder Alkoxy mit je 1 oder 2 Kohlenstoffatomen substituiertes Phenylalkyl mit 1 bis 4 Kohlenstoffatomen im Alkylteil oder $H-(-OC_2H_4-)-_n$sind, worin n 1 oder 2 ist,

$Z_1$ gegebenenfalls mit Alkyl oder Alkoxy mit je 1 bis 4 Kohlenstoffatomen, Hydroxyl, Cyano, Nitro oder Halogen substituiertes Phenylen ist, wobei der Phenylenring 1 oder 2 der Substituenten enthalten kann.

Wertvolle Verbindungen der Formel (1) sind solche, worin

$B_1$ Wasserstoff, gegebenemfalls mit Hydroxyl, Cyano, Carbalkoxy mit 2 bis 9 Kohlenstoffatomen oder Carbonamido substituiertes Alkyl mit 1 bis 4 Kohlenstoffatomen, Cyclohexyl, Benzyl, Phenyläthyl oder gegebenenfalls mit Methoxy oder Äthoxy substituiertes Phenyl ist, und

$R_1$, $R_2$ und $R_3$ unabhängig voneinander gegebenenfalls mit Hydroxyl substituiertes Alkyl mit je 1 bis 4 Kohlenstoffatomen, Benzyl, Phenyläthyl oder $H-(-OC_2H_4-)-_n$ sind, worin n 1 oder 2 ist

$A_1$ gegebenenfalls mit Alkyl oder Alkoxy mit je 1 bis 4 Kohlenstoffatomen, Hydroxyl oder Halogen substituiertes Phenyl ist wobei der Phenylring 1 oder 2 der Substituenten tragen kann, und

$Z_1$ gegebenenfalls mit Alkyl oder Alkoxy mit je 1 bis 4 Kohlenstoffatomen, Hydroxyl, Cyano, Nitro oder Halogen substituiertes Phenylen ist, wobei der Phenylenring 1 oder 2 der Substituenten enthalten kann.

Interessant sind des weiteren solche Verbindungn der Formel (1), worin

$B_1$ Wasserstoff gegebenenfalls mit Hydroxyl, Cyano, Carbalkoxy mit 2 bis 5 Kohlenstoffatomen oder Carbonamido substituiertes Alkyl mit 1 oder 2 Kohlenstoffatomem, Benzyl oder Phenyl ist, und

$R_1$, $R_2$ und $R_3$ unabhängig voneinander Alkyl mit je 1 oder 2 Kohlenstoffatomen, Benzyl oder $H-(-OC_2H_4-)-_n$ sind, worin n 1 oder 2 ist,

$A_1$ gegebenenfalls mit Alkyl oder Alkoxy mit je 1 bis 4 Kohlenstoffatomen, Hydroxyl oder Halogen substituiertes Phenyl ist, wobei der Phenylring 1 oder 2 der Substituenten tragen kann, und

$Z_1$ gegebenenfalls mit Alkyl oder Alkoxy mit je 1 bis 4 Kohlenstoffatomen, Hydroxyl, Cyano, Nitro oder Halogen substituiertes Phenylen ist, wobei der Phenylenring 1 oder 2 der Substituenten enthalten kann,

und besonders solche der Formel (1), worin

$A_1$ gegebenenfalls mit Alkyl oder Alkoxy mit je 1 oder 2 Kohlenstoffatomen oder Chlor substituiertes Phenyl ist, wobei der Phenylring 1 oder 2 der Substituenten tragen kann,

$Z_1$ gegebenenfalls mit Methoxy, Hydroxyl oder Chlor substituiertes 1,4-Phenylen ist, wobei der Phenylenring 1 oder 2 der Substituenten tragen kann, und

$B_1$, $R_1$, $R_2$ und $R_3$ die oben angegebenen Bedeutungen haben.

Besonders wertvolle Verbindungen sind solche der Formel (1), worin

$A_1$ gegebenenfalls mit Methyl, Methoxy oder Chlor substituiertes Phenyl ist, wobei der Phenylring 1 oder 2 der Substituenten tragen kann,

$R_1$, $R_2$ und $R_3$ unabhängig voneinander Methyl, Benzyl oder $HO-C_2H_4-$ sind,

$B_1$ Wasserstoff, gegebenenfalls mit Hydroxyl, Cyano, Carbalkoxy mit 2 bis 5 Kohlenstoffatomen oder Carbonamido substituiertes Alkyl mit 1 oder 2 Kohlenstoffatomen, Benzyl oder Phenyl ist, und

$Z_1$ gegebenenfalls mit Methoxy, Hydroxyl oder Chlor substituiertes 1,4-Phenylen ist, wobei der Phenylenring 1 oder 2 der Substituenten tragen kann.

Die Verbindungen der Formel (1) können hergestellt werden indem man die Verbindung der Formel

(8) 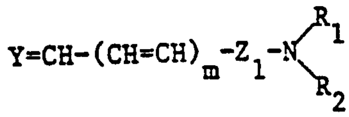 ,

worin
Y Sauerstoff oder =N-D ist, wobei D einen aromatischen Rest bedeutet, und $Z_1$, $R_1$, $R_2$ und m die oben angegebene Bedeutung haben,
mit einem Hydrazin der Formel

(9) 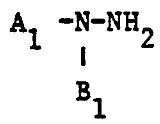 ,

worin
$A_1$ und $B_1$ die oben angegebenen Bedeutungen haben, kondensiert und anschliessend das zu $Z_1$ benachbarte Stickstoffatom quaterniert.

Bevorzugte Verbindungen der Formel (8) sind z. B.
4-Dimethylamimobenzaldehyd,
4-Diäthylaminobenzaldehyd, oder
4-Dimethylaminozimtaldehyd.

Geeignete Hydrazine der Formel (9) sind z. B.
N-Phenyl-N-methylhydrazin,
N-Phenyl-N-äthylhydrazin,
N-Phenyl-N-benzylhydrazin,
N-(4-Methylphenyl)-N-methylhydrazin,
N-(4-Methoxyphenyl)-N-methylhydrazin oder
N-(4-Chlorphenyl)-N-methylhydrazin.

In einem weiteren Verfahren werden Hydrazonderivate der Formel (1) durch Umsetzung der Verbindung der Formel (8) mit einem Hydrazin der Formel

(10) 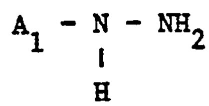 ,

worin
$A_1$ die oben angegebene Bedeutung hat, anschliessender Substitution des zu $A_1$ geminalen Wasserstoffatoms durch $B_1$ und nachfolgender Quaternierung des zu $Z_1$ benachbarten Stickstoffatoms hergestellt.

Substitution und Quaternierung können sowohl nacheinander als auch in einem Schritt erfolgen.

Ferner könnem Verbindungen der Formel (1) direkt durch Reaktion der quaternierten Verbindung

(11) 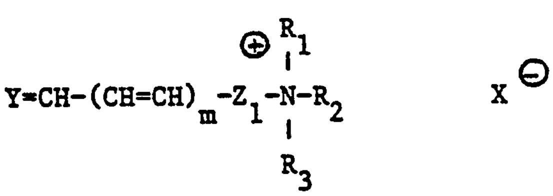 ,

worin
Y, $Z_1$, $R_1$, $R_2$, $R_3$, $X^{\ominus}$ und m die oben angegebenen Bedeutungen haben, mit einem Hydrazin der Formel (9) hergestellt werden.

Die Kondensation der Verbindungen (8) und (9), (8) und (10) sowie (9) und (11) erfolgt zweckmässigerweise in einem Lösungsmittel, bevorzugt in einem Alkohol wie z. B. Methanol oder Äthanol. Geeignete

Quaternierungsmittel sind z. B. Dimethylsulfat, Diäthylsulfat, p-Toluolsulfonsäureester, Benzylbromid oder Methyljodid. Die Quaternierung erfolgt vorzugsweise in einem Lösungsmittel wie z. B. Aceton, Methyläthylketon, Ligroin, Chloroform, Benzol, Toluol oder Chlorbenzol.

Die Verbindungen der Formel (1) sind wasserlösliche Substanzen, die ähnlich wie basische Farbstoffe von natürlichen oder synthetischen hochmolekularen Verbindungen mit sauren Gruppen fest gebunden werden. Die Verbindungen der Formel (1) absorbieren ultraviolette und sichtbare Strahlung im Wellenlängenbereich zwischen etwa 320 und 450 mm. Sie sind deshalb als UV-Absorber geeignet.

Eine wichtige Anwendung, für welche die Verbindungen der Formel (1) besonders geeignet sind, ist die "Einfärbung" von sauren Photopolymeren mit UV-absorbierenden Stoffen.

Photopolymere werden häufig als Masken zur Herstellung von Kopien benützt. Photopolymere, die für derartige Zwecke verwendbar sind, müssen für photochemisch wirksame Strahlung, vorzugsweise im ultravioletten bis blauen Bereich undurchlässig sein.

Geeignete Photopolymere sind z. B. die gemäss der Europäischen Patentanmeldung 21 019 hergestellten Dimethylmaleinimidgruppen enthaltenden Polymere, die in der Polymerkette freie saure Gruppen, z. B. von eingebauten Acryl- oder Methacrylsäuremolekülen, enthalten. Gemäss der erwähnten Patentanmeldung können solche Photopolymere nach erfolgter Belichtung und gleichzeitig mit oder nach der mit alkalischen wässerigen Lösungen erfolgenden Entwicklung mit kationischen Farbstoffen eingefärbt werden.

Anstelle der üblichen kationischen Farbstoffe können nun auch kationische UV-Absorber für den gleichen Zweck mit Erfolg verwendet werden. Durch diese Behandlung wird das Photopolymer an den belichteten Stellen nach deren Entwicklung und Einfärbung undurchlässig für ultraviolette Strahlung und eignet sich damit zur Herstellung von Kopien oder als Maske, z. B. für die Herstellung von gedruckten Schaltungen, oder Halbleiterelementen mit integrierten Schaltkreisen, sofern zur Herstellung dieser Produkte bevorzugt im UV-Bereich empfindliche Photoschichten verwendet werden. Die UV-Absorber der Formel (1) enthaltenden Bildbereiche können zu diesem Zweck auch mit weiteren, im sichtbaren Spektralbereich absorbierenden Farbstoffen für das Auge sichtbar gemacht werden.

Die nachfolgenden Beispiele sollen Herstellung und Verwendung der kationischen UV-Absorber erläutern. Teile und Prozente beziehen sich auf das Gewicht.

**Herstellungsbeispiele**

**Beispiel 1**

61 Teile 4-Dimethylaminobenzaldehyd werden in 450 Teilen Äthylalkohol und 45 Teilen Essigsäure bei 40°C gelöst. Unter Rühren tropft man langsam 50 Teile N-Methyl-N-phenylhydrazin, gelöst in 250 Teilen Äthanol und 250 Teilen Wasser, hinzu. In quantitativer Ausbeute erhält man die Verbindung der Formel

(101) 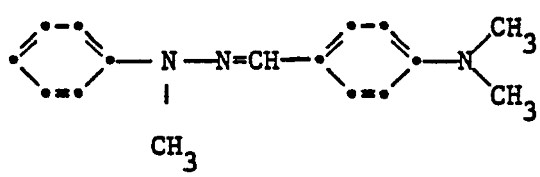

vom Schmelzpumkt 144-146°C.

Analyse:

| | % ber. | % gef. |
|---|---|---|
| C | 75,86 | 75,67 |
| H | 7,56 | 7,53 |
| N | 16,59 | 16,76 |

Die anschliessende Quaternierung erfolgt durch Erhitzen einer Suspension von 10 Teilen der Verbindung (101) in 40 Teilen Methyläthylketon auf 70°C und darauffolgender Zugäbe von 5 Teilen Dimethylsulfat. Nach etwa 10 Minuten kristallisiert die Verbindung der Formel

(102) 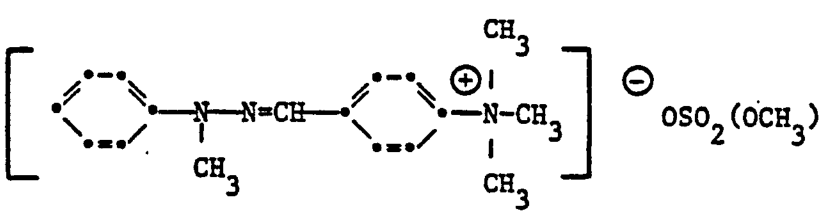

aus.

Der Schmelzpunkt beträgt 144-150° C, λ max=355 nm und ε=25000.

Analyse:

| | % ber. | %gef. |
|---|---|---|
| C | 56.97 | 56.70 |
| H | 6.64 | 6.60 |
| N | 11.07 | 11.23 |

**Beispiel 2**

Gemäss Beispiel 1 wird durch Umsetzung von 4-Dimethylamino-benzaldehyd mit Phenylhydrazin die Verbindung der Formel

(105) 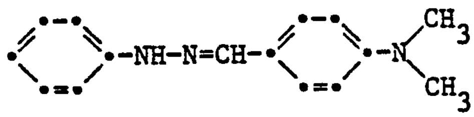

hergestellt.

Der Schmelzpunkt beträgt 144-147,5° C; λ max=360 nm und ε=35000.

Analyse:

| | % ber. | % gef. |
|---|---|---|
| C | 55,87 | 55,88 |
| H | 6,34 | 6,38 |
| N | 11,49 | 11,48 |

24 Teile der Verbindung der Formel (105) werden in 85 Teilen Methyläthylketon gelöst und nach Zugabe von 11 Teilen Dimethylsulfat 4 Stunden auf 70° C erhitzt. Man erhält 34 Teile der quaternierten Verbindung der Formel

(114) 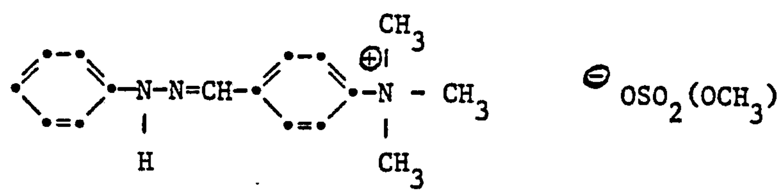

vom Schmelzpunkt 197-198° C; λ max=341 nm und ε=20000.

Analyse:

| | C | H | N |
|---|---|---|---|
| % ber. | 55,87 | 6,34 | 11,49 |
| % gef. | 55,76 | 6,38 | 11,45 |

In analoger Weise lässt sich 4-Dimethylaminozimtaldehyd mit N-Phenyl-N-Methylhydrazin zu dem Hydrazon der Formel

(110) 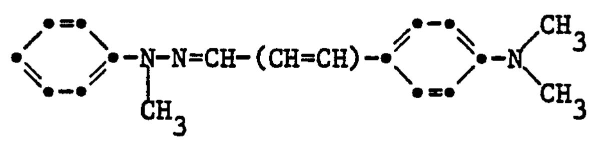

umsetzen.

Schmelzpunkt 168-170° C.

NMR-Daten: Singlett bei 2,9 ppm 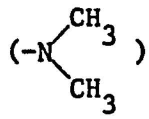

3,25 ppm 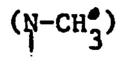

6,7-7,5 ppm (12 Protonen)

Die Verbindung der Formel (110) wird wie beschrieben zu der entsprechenden quaternierten Verbindungen der Formel (113) umgesetzt.

(113) 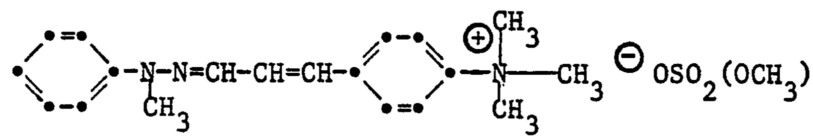

Schmelzpunkt 187-190° C, $\varepsilon=30000$, $\lambda_{max}=365$ mm.
NMR-Daten: Singlett:
3,3 ppm ($N\text{-}CH_3$)
3,4 ppm$\ominus$ $OSO_2(OCH_3)$
3,6 ppm$\oplus N\text{-}(CH_3)_3$

6,1-7,2 ppm (12 Protonen)

**Anwendungsbeispiele**

**Beispiel 3**

Auf einen Polyesterträger wird eine lichtempfindliche Schicht von 2 µ Dicke bestehend aus einem Polymeren von 20 % Methacrylsäure und 80 % N-(2-Methacryloyloxyäthyl)-dimethylmaleinimid welches zuvor mit einer Thioxanthonverbindung sensibilisiert wurde, aufgebracht.

Ein Streifen des beschichteten Materials wird hinter einer transparenten Vorlage bildmässig belichtet und danach durch Eintauchen in eine 2 %-ige Lösung der Verbindung der Formel (102) in einer 0,25-molaren wässerigen Lösung von Trinatriumphosphat entwickelt. Anschliessend wird 30 Sekunden in Wasser gewaschen und danach getrocknet.

Man erhält ein UV-absorbierendes Negativbild, dessen optische Dichte, gemessen bei $\lambda=355$ nm, nach einer Entwicklungszeit von 20 Sekunden über 5,0 beträgt.

**Beispiel 4**

Auf einen Polyesterträger wird eine 2 µ dicke Schicht eines photovernetzenden Polymeren aufgebracht, das durch Copolymerisation von 20 % Methacrylsäure, 20 % Äthylacrylat und 60 % N-(2-Methacryloyloxyäthyl)-dimethylmaleinimid (inhärente Viskosität 0,24) erhalten und mit einer Thioxanthonverbindung sensibilisiert worden ist.

Wie in Beispiel 3 wird ein Streifen dieses Materials hinter einer transparenten Vorlage bildmässig belichtet und danach 20 Sekunden in eine Lösung getaucht, die wie folgt hergestellt wird:
22 g der Verbindung der Formel (102),
25 g des Farbstoffs CI 45055 und
22 g des Azofarbstoffs der Formel

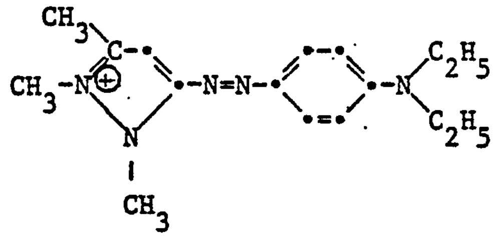

$^{\ominus}OSO_2(OCH_3)$

werden in 2000 g einer 0,25-molaren Lösung von Trinatriumphosphat aufgelöst. Durch Zusatz von 30 %-iger Natronlauge stellt man die Lösung auf einen pH-Wert von 13,0 ein.

Danach wird mit Wasser gewaschen und getrocknet. Das Material besitzt an den Bildstellen im ganzen Spektralbereich zwischen 350 und 500 nm eine optische Dichte von mindestens 3,0.

**Beispiel 5**

Ein Streifen des im Beispiel 3 verwendeten Materials wird wie zuvor belichtet und danach durch Eintauchen in eine 5 %-ige Lösung von Natriumcarbonat entwickelt und anschliessend mit Wasser gewaschen. Anschliessend wird der Streifen durch Eintauchen in eine Lösung von

1,5 Gew.-% der Verbindung der Formel (102)
4,0 " des Farbstoffs CI 48055
0,75 " des Farbstoffs CI 48035
0,75 " des Farbstoffs CI 11055 (s) und
0,25 " Natriumcarbonat

20 Sekunden angefärbt und anschliessend mit Wasser ausgewaschen. Nach dem Trocknen beträgt die optische Dichte an den Bildstellen im gesamten Spektralbereich zwischen 350 und 500 nm mindestens 3,0.

**Beispiel 6**

Auf einen 0,1 mm dicken Polyesterträger wird eine 3 μm dicke Schicht eines photovernetzbaren Copolymeren aufgebracht, das durch Copolymerisation von 15 % Methacrylsäure, 25 % Äthylacrylat und 60 % N-(2-Methacryloyloxyäthyl)-dimethylmaleinimid (inh. Viskosität = 0,29 dl/g; 0,5 % in Methyläthylketon/Methylcellosolve 1 : 1; 25°C) hergestellt und mit 8 % (bezogen auf Feststoffgehalt) einer Thioxanthonverbindung sensibilisiert worden ist.

Vier Proben dieses Materials werden belichtet und 20 Sekunden in eine bewegte, 5 %-ige wässrige Natriumbicarbonatlösung getaucht, die mit 30 %-iger Natronlauge auf einen pH-Wert von 9,7 eingestellt wurde und eine Temperatur von 35°C besitzt. Die Proben werden anschliessend in deionisiertem Wasser 20 Sekunden bei Raumtemperatur gespült.

Je eine Probe wird durch Eintauchen in verschiedene Farbstofflösungen (A, B, C und D) 20 Sekunden gefärbt. Die Temperatur der Farbstofflösungen beträgt in jedem Falle 20°C. Die Farbstofflösungen werden wie folgt hergestellt:

Man löst 2 g eines kationischen UV-Absorbers in 100 ml einer Pufferlösung, die durch Lösen von 10 g $KH_2PO_4$ in 2490 g deionisiertem Wasser hergestellt wurde. Der pH-Wert dieser Lösung wurde mit Hilfe 30 %-iger wässriger Kalilauge auf 6,0 bzw. 7,0 eingestellt. Als kationische UV-Absorber werden die Verbindungen der Formeln (102), (113), (114) sowie die Verbindung der Formel

(117)

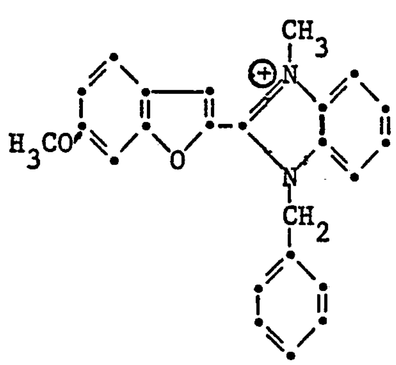

$CH_3OSO_3^{\ominus}$ (als Vergleich)

eingesetzt.

Verbindung (117) ist ein handelsüblicher optischer Aufheller. Die gefärbten Proben werden 30 Sekunden in Wasser gewaschen und getrocknet. Mit einem UV-Spektralphotometer wird die optische Dichte der Proben ermittelt. Die Ergebnisse sind in Tabelle 3 zusammengestellt.

**Tabelle**

| Farbstofflösung | Dichte bei pH 6 (bei $\lambda_{max}$) | Dichte bei pH 7 | Optische Dichte $\geqslant 3{,}0$ bei pH 7 von |
|---|---|---|---|
| A (mit Verb.(102)) | >5,0 (350 nm) | >5,0 | 315-385 nm |
| B (mit Verb.(113)) | >5,0 (375 nm) | >5,0 | 340-410 nm |
| C (mit Verb.(114)) | >5,0 (360 nm) | >5,0 | 320-400 nm |
| D (mit Verb.(117)) als Vergleich | >3,8 (355 nm) | 4,2 | 330-380 nm |

Aus der Tabelle ist ersichtlich, dass die erfindungsgemässen Verbindungen der Formeln (102), (113) und (114) sowohl auf Grund ihrer optischen Dichte als auch des wirksamen Spektralbereiches wesentlich bessere Anfärbeeigenschaften aufweisen als die Vergleichsverbindung der Formel (117).

**Patentansprüche**

1. Verfahren zur Herstellung einer UV-Strahlung absorbierenden Maske, bei dem man eim saures photopolymerisierbares Polymer bildmässig belichtet, anschliessend entwickelt und an den belichteten Stellen gleichzeitig oder nach der Entwicklung mit einem kationischen Hydrazonderivat der folgenden Formel (1) behandelt,

(1) 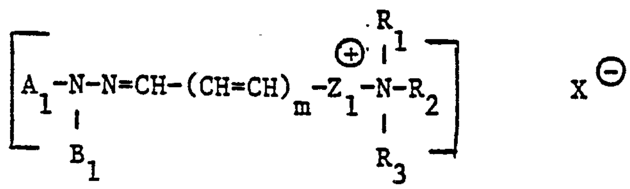

worin

$A_1$ ein gegebenenfalls mit Alkyl oder Alkoxy mit je 1 bis 4 Kohlenstoffatomen, Hydroxyl oder Halogen substituierter aromatischer Rest,

$B_1$ Wasserstoff, gegebenenfalls mit Methoxy, Hydroxyl, Cyano, Halogen, Carbalkoxy mit 2 bis 9 Kohlenstoffatomen oder Carbonamido substituiertes Alkyl mit 1 bis 8 Kohlenstoffatomen, gegebenenfalls mit Alkyl oder Alkoxy mit je 1 oder 2 Kohlenstoffatomen substituiertes Cycloalkyl mit 5 oder 6 Ringkohlenstoffatomen, gegebenenfalls im Arylteil mit Alkyl oder Alkoxy mit je 1 bis 4 Kohlenstoffatomen substituiertes Aralkyl mit 1 bis 4 Kohlenstoffatomen im Alkylteil, Naphthyl oder gegebenenfalls mit Alkoxy mit 1 bis 4 C-Atomen substituiertes Phenyl ist,

$R_1$, $R_2$ und $R_3$ unabhängig voneinander gegebenenfalls mit Methoxy, Hydroxy, Cyano, Halogen oder Carbonamido substituiertes Alkyl mit je 1 bis 8 Kohlenstoffatomen, gegebenenfalls mit Methyl oder Methoxy substituiertes Cycloalkyl mit 5 oder 6 Ringkohlenstoffatomen, gegebenenfalls im Phenylteil mit Alkyl oder Alkoxy mit 1 bis 4 C-Atomen substituiertes Phenylalkyl mit 1 bis 4 Kohlenstoffatomen im Alkylteil oder Polyoxyalkylen sind,

$Z_1$ gegebenenfalls mit Hydroxyl, Cyano, Nitro, Halogen, Alkyl oder Alkoxy mit je 1 bis 4 Kohlenstoffatomen substituiertes Arylen,

$X^{\ominus}$ ein Anion einer gegebenenfalls mehrbasischen Mineralsäure, und

m 0, 1, 2 oder 3 sind.

2. Verfahren nach Anspruch 1, dadurch gekennzeichnet, dass $A_1$ als aromatischer Rest Phenyl darstellt.

3. Verfahren nach Anspruch 1, dadurch gekennzeichnet, dass

$A_1$ gegebenenfalls mit Alkyl oder Alkoxy mit je 1 bis 4 Kohlenstoffatomen, Hydroxyl oder Halogen substituiertes Phenyl, wobei der Phenylring 1 oder 2 der Substituenten tragen kann,

$B_1$ Wasserstoff, gegebenfalls mit Methoxy, Hydroxyl, Cyano, Chlor, Carbalkoxy mit 2 bis 9 Kohlenstoffatomen oder Carbonamido substituiertes Alkyl mit 1 bis 4 Kohlenstoffatomen, gegebenenfalls mit Methyl oder Methoxy substituiertes Cyclohexyl, gegebenenfalls mit Alkyl oder Alkoxy mit je 1 oder 2 Kohlenstoffatomen im Arylteil substituiertes Aralkyl mit 1 bis 4 Kohlenstoffatomen im Alkylteil oder gegebenenfalls mit Alkoxy mit 1 oder 2 Kohlenstoffatomen substituiertes Phenyl ist,

$R_1$, $R_2$ und $R_3$ unabhängig voneinander gegebenenfalls mit Methoxy, Hydroxyl, Cyano, Chlor oder Carbonamido substituiertes Alkyl mit je 1 bis 4 Kohlenstoffatomen, gegebenenfalls mit Methyl oder Methoxy

substituiertes Cyclohexyl, gegebenenfalls im Phenylteil mit Alkyl oder Alkoxy mit je 1 oder 2 Kohlenstoffatomen substituiertes Phenylalkyl mit 1 bis 4 Kohlenstoffatomen im Alkylteil oder $H\text{-}(\text{-}OC_2H_4\text{-})\text{-}_n$ sind, worin n 1 oder 2 ist,

$Z_1$ gegebenenfalls mit Alkyl oder Alkoxy mit je 1 bis 4 Kohlenstoffatomen, Hydroxyl, Cyano, Nitro oder Halogen substituiertes Phenylen ist, wobei der Phenylenring 1 oder 2 der Substituenten enthalten kann.

4. Verfahren nach Anspruch 3, dadurch gekennzeichnet, dass

$B_1$ Wasserstoff, gegebenenfalls mit Hydroxyl, Cyano, Carbalkoxy mit 2 bis 9 Kohlenstoffatomen oder Carbonamido substituiertes Alkyl mit 1 bis 4 Kohlenstoffatomen, Cyclohexyl, Benzyl, Phenyläthyl oder gegebenenfalls mit Methoxy oder Äthoxy substituiertes Phenyl ist,

$R_1$, $R_2$ und $R_3$ unabhängig voneinander gegebenenfalls mit Hydroxyl substituiertes Alkyl mit je 1 bis 4 Kohlenstoffatomen, Benzyl, Phenyläthyl oder $H\text{-}(\text{-}OC_2H_4\text{-})\text{-}_n$ sind, worin n 1 oder 2 ist, und

$A_1$ und $Z_1$ die in Anspruch 3 angegebenen Bedeutungen haben.

5. Verfahren nach Anspruch 4, dadurch gekennzeichnet, dass

$B_1$ Wasserstoff, gegebenenfalls mit Hydroxyl, Cyano, Carbalkoxy mit 2 bis 5 Kohlenstoffatomen oder Carbonamido substituiertes Alkyl mit 1 oder 2 Kohlenstoffatomen, Benzyl oder Phenyl ist,

$R_1$, $R_2$ und $R_3$ unabhängig voneiander Alkyl mit je 1 oder 2 Kohlenstoffatomen, Benzyl oder $H\text{-}(\text{-}OC_2H_4\text{-})\text{-}_n$ sind, worin n 1-oder 2 ist, und

$A_1$ und $Z_1$ die in Anspruch 3 angegebenen Bedeutungen haben.

6. Verfahren nach Anspruch 5, dadurch gekennzeichnet, dass

$A_1$ gegebenenfalls mit Alkyl oder Alkoxy mit je 1 oder 2 Kohlenstoffatomen oder Chlor substituiertes Phenyl ist, wobei der Phenylring 1 oder 2 der Substituenten tragen kann,

$Z_1$ gegebenenfalls mit Methoxy, Hydroxyl oder Chlor substituiertes 1,4-Phenylen ist, wobei der Phenylenring 1 oder 2 der Substituenten tragen kann, und

$B_1$, $R_1$, $R_2$ und $R_3$ die in Anspruch 5 angegebenen Bedeutungen haben.

7. Verfahren nach Anspruch 6, dadurch gekennzeichnet, dass

$A_1$ gegebenenfalls mit Methyl, Methoxy oder Chlor substituiertes Phenyl ist, wobei der Phenylring 1 oder 2 der Substituenten tragen kann,

$R_1$, $R_2$ und $R_3$ unabhängig voneianander Methyl, Benzyl oder $HO\text{-}C_2H_4$-sind, und

$Z_1$ und $B_1$ die in Anspruch 6 angegebenen Bedeutungen haben.

## Claims

1. Process for the preparation of a UV-absorbing mask, in which an acidic photopolymerisable polymer is exposed imagewise, subsequently developed and the exposed areas are treated simultaneously or after the development with a cationic hydrazone derivative of the following formula (1),

(1) 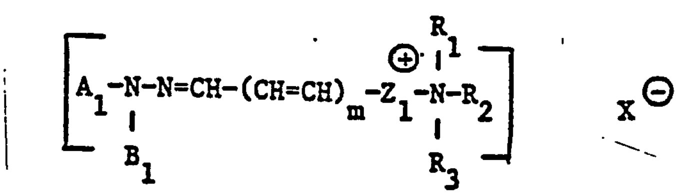

in which $A_1$ is an aromatic radical which is unsubstituted or substitued by alkyl or alkoxy each having 1 to 4 carbon atoms, or by hydroxyl or halogen, $B_1$ is hydrogen, alkyl having 1 to 8 carbon atoms which is unsubstituted or substituted by methoxy, hydroxyl, cyano, halogen, carbalkoxy having 2 to 9 carbon atoms or carbonamido, cycloalkyl having 5 or 6 ring carbon atoms which is unsubstituted or substituted by alkyl or alkoxy having 1 or 2 carbon atoms, aralkyl having 1 to 4 carbon atoms in the alkyl moiety which is unsubstituted or substituted in the aryl moiety by alkyl or alkoxy each having 1 to 4 carbonatoms, naphthyl or phenyl which is unsubstituted or substituted by alkoxy having 1 to 4 C-atoms, $R_1$, $R_2$ and $R_3$ independently of one another are alkyl having in each case 1 to 8 carbon atoms which is unsubstituted or substituted by methoxy, hydroxyl, cyano, halogen or carbonamido, cycloalkyl having 5 or 6 ring carbon atoms which is unsubstitued or substituted by methyl or methoxy, phenylalkyl having 1 to 4 carbon atoms in the alkyl moiety which is unsubstituted or substituted in the phenyl moiety by alkyl or alkoxy having 1 to 4 C-atoms, or polyoxyalkylene, $Z_1$ is arylene which is unsubstituted or substituted by hydroxyl, cyano, nitro, halogen, alkyl or alkoxy each having 1 to 4 carbon atoms, $X^{\oplus}$ is an anion of a mineral acid which may be polybasic, and m is 0, 1, 2 or 3.

2. Process according to Claim 1, characterized in that $A_1$ as an aromatic radical is phenyl.

3. Process according to Claim 1, characterized in that $A_1$ is phenyl which is unsubstituted or substituted by alkyl or alkoxy each having 1 to 4 carbon atoms or by hydroxyl or halogen, where the phenyl ring can carry 1 or 2 of the substituents, $B_1$ is hydrogen, alkyl having 1 to 4 carbon atoms which is unsubstituted or substituted by methoxy, hydroxyl, cyano, chlorine, carbalkoxy having 2 to 9 carbon atoms or carbonamido, cyclohexyl which is unsubstituted or substituted by methyl or methoxy, aralkyl having 1 to 4 carbon atoms within the alkyl moiety

which is unsubstituted or substituted by alky or alkoxy each having 1 or 2 carbon atoms in the aryl moiety, or phenyl which is unsubstituted or substituted by alkoxy having 1 or 2 carbon atoms, $R_1$, $R_2$ and $R_3$ independently of one another are alkyl having in each case 1 to 4 carbon atoms which is unsubstituted or substituted by methoxy, hydroxyl, cyano, chlorine or carbonamido, cyclohexyl which is unsubstituted or substituted by methyl or methoxy, phenylalkyl having 1 to 4 carbon atoms in the alkyl moiety which is unsubstituted or substituted in the phenyl moiety by alkyl or alkoxy each having 1 or 2 carbon atoms or $H\text{-}(OC_2H_4)\text{-}_n$, wherein n is 1 or 2, $Z_1$ is phenylene which is unsubstituted or substituted by alkyl or alkoxy each having 1 to 4 carbon atoms, by hydroxyl, cyano, nitro or halogen, where the phenyl ring can contain 1 or 2 of the substitutents.

4. Process according to Claim 3, characterized in that $B_1$ is hydrogen, alkyl having 1 to 4 carbon atoms which is unsubstituted or substituted by hydroxyl, cyano, carbalkoxy having 2 to 9 carbon atoms or carbonamido, or is cyclohexyl, benzyl, phenylethyl or phenyl which is unsubstituted or substituted by methoxy or ethoxy, $R_1$, $R_2$ and $R_3$ independently of one another are alkyl having in each case 1 to 4 carbon atoms which is unsubstituted or substituted by hydroxyl, or are benzyl, phenylethyl or $H\text{-}(OC_2H_4)\text{-}_n$, wherein n is 1 or 2 and $A_1$ and $Z_1$ are as defined in Claim 3.

5. Process according to Claim 4, characterized in that $B_1$ is hydrogen, alkyl having 1 or 2 carbon atoms which is unsubstituted or substituted by hydroxyl, cyano, carbalkoxy having 2 to 5 carbon atoms or carbonamido, or is benzyl or phenyl, $R_1$, $R_2$ and $R_3$ independently of one another are alkyl having in each case 1 or 2 carbon atoms, benzyl or $H\text{-}(OC_2H_4)\text{-}_n$, wherein n is 1 or 2, and $A_1$ and Z are as defined in Claim 3.

6. Process according to Claim 5, characterized in that $A_1$ is phenyl which is unsubstituted or substituted by alkyl or alkoxy each having 1 or 2 carbon atoms or by chlorine, where the phenyl ring can carry 1 or 2 of the substitutents, $Z_1$ is 1,4-phenylene which is unsubstituted or substituted by methoxy, hydroxyl or chlorine, where the phenyl ring can carry 1 or 2 of the substituents, and $B_1$, $R_1$, $R_2$ and $R_3$ are as defined in Claim 5.

7. Process according to Claim 6, characterized in that $A_1$ is phenyl which is unsubstituted or substituted by methyl, methoxy or chlorine, where the phenyl ring can carry 1 or 2 of the substituents, $R_1$, $R_2$ and $R_3$ independently of one another are methyl, benzyl or $HO\text{-}C_2H_4\text{-}$, and $Z_1$ and $B_1$ are as defined in Claim 6.

## Revendications

1. Procédé pour réaliser un masque absorbant un rayonnement ultra-violet, procédé selon lequel on expose à la lumière, conformément à une image un polymère photopolymérisable acide, puis on le développe et, en même temps que le développement ou après celui-ci, on effectue un traitement, affectant les endroits touchés par la lumière, avec un dérivé cationique d'une hydrazone qui répond à la formule générale 1:

(1) 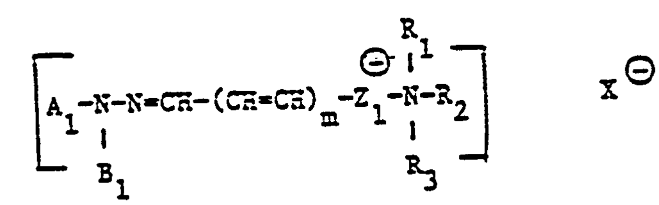

dans laquelle

$A_1$ représente un radical aromatique éventuellement porteur d'un alkyle contenant de 1 à 4 atomes de carbone, d'un alcoxy contenant de 1 à 4 atomes de carbone, d'un hydroxy ou d'un halogène,

$B_1$ représente:

- l'hydrogène,
- un alkyle en $C_1$-$C_8$ qui porte éventuellement un méthoxy, un hydroxy, un cyano, un halogène, un alcoxycarbonyle en $C_2$-$C_9$ ou un carbonylamino,
- un cycloalkyle qui contient 5 ou 6 atomes de carbone dans son cycle et qui porte éventuellement un radical alkyle ou un radical alcoxy contenant chacun 1 ou 2 atomes de carbone,
- un aralkyle dont la partie alkyle contient de 1 à 4 atomes de carbone et dont la partie aryle porte éventuellement un radical alkyle ou un radical alcoxy contenant chacun de 1 à 4 atomes de carbone,
- un naphtyle ou
- un phényle éventuellement porteur d'un alcoxy contenant de 1 à 4 atomes de carbone,

$R_1$, $R_2$ et $R_3$ représentent chacun, indépendamment les uns des autres:

- un alkyle en $C_1$-$C_8$ qui porte éventuellement un méthoxy, un hydroxy, un cyano un halogène ou un carbonylamino,
- un cycloalkyle dont le cycle contient 5 ou 6 atomes de carbone et qui porte éventuellement un méthyle ou un méthoxy,
- un phénylalkyle dont la partie alkyle contient de 1 à 4 atomes de carbone et dont la partie phényle porte éventuellement un alkyle en $C_1$-$C_4$ ou un alcoxy en $C_1$-$C_4$, ou
- un poly-oxy-alkylène,

$Z_1$ représente un arylène éventuellement porteur d'un hydroxy, d'un cyano, d'un nitro, d'un halogpne, d'un alkyle en $C_1$-$C_4$ ou d'un alcoxy en $C_1$-$C_4$,

$X^{\ominus}$ représente un anion d'un acide minéral comportant éventuellement plusieurs fonctions acidas et m est égal à 0, à 1, à 2 ou à 3.

2. Procédé selon la revendication 1 caractérisé en ce que $A_1$ en tant que radical aromatique, est un radical phényle.

3. Procédé selon la revendication 1 caractérisé en ce que:

$A_1$ représente un radical phényle qui peut porter 1 ou 2 substituants pris dans l'ensemble constitué par les alkyles en $C_1$-$C_4$, les alcoxy en $C_1$-$C_4$, l'hydroxy et les halogènes,

$B_1$ représente:

- l'hydrogène,
- un alkyle qui contient de 1 à 4 atomes de carbone et qui porte éventuellement un méthoxy un hydroxy un cyano, un chlore, un alcoxycarbonyle en $C_2$-$C_9$ ou un carbonylamino,
- un cyclohexyle éventuellement porteur d'un méthyle ou d'un méthoxy,
- un aralkyle dont la partie alkyle contient de 1 à 4 atomes de carbone et dont la partie aryle porte éventuellement un alkyle à 1 ou 2 atomes de carbone ou un alcoxy à 1 ou 2 atomes de carbone, ou
- un phényle éventuellement porteur d'un alcoxy à 1 ou 2 atomes de carbone,

$R_1$, $R_2$ et $R_3$ représentent chacun, indépendemment les uns des autres:

- un alkyle qui contient de 1 à 4 atomes de carbone et qui porte éventuellement un méthoxy, un hydroxy, un cyano, un chlore ou un carbonylamino,
- un cyclohexyle éventuellement porteur d'un méthyle ou d'un méthoxy,
- un phénylalkyle dont la partie alkyle contient de 1 à 4 atomes de carbone et dont la partie phényle porte éventuellement un alkyle à 1 à 2 atomes de carbone ou un alcoxy à 1 ou 2 atomes de carbone ou
- un radical $H$-$(-OC_2H_4-)$-$_n$ dans lequel n est égal à 1 ou à 2,

et

$Z_1$ représente un radical phénylène qui porte éventuellement un ou deux substituants pris dans l'ensemble constitué par les alkyles en $C_1$-$C_4$, les alcoxy en $C_1$-$C_4$, l'hydroxy, le cyano, le nitro et les halogènes.

4. Procédé selon la revendication 3 caractérisé en ca que:

$B_1$ représente l'hydrogène, un alkyle en $C_1$-$C_4$ éventuellement porteur d'un hydroxy, d'un cyano, d'un alcoxycarbonyle en $C_2$-$C_9$ ou d'un carbonylamino, un cyclohexyle, un benzyle, un phényléthyle ou un phényle éventuellement porteur d'un méthoxy ou d'un éthoxy,

$R_1$, $R_2$ et $R_3$ représentent chacun, indépendamment les uns des autres,un alkyle en $C_1$-$C_4$ éventuellement porteur d'un hydroxy, un benzyle, un phényléthyle ou un radical $H$-$(OC_2H_4)_n$ - dans lequel n est égal à 1 ou à 2,

et

$A_1$ et $Z_1$ ont les significations données à la revendication 3.

5. Procédé selon la revendication 4 caractérisé en ce que:

$B_1$ représente l'hydrogène, un alkyle à 1 ou 2 atomes de carbone, éventuellement porteur d'un hydroxy, d'un cyano, d'un alcoxycarbonyle en $C_2$-$C_5$ ou d'un carbonylamino, un benzyle ou un phényle,

$R_1$, $R_2$ et $R_3$ représentent chacun, indépendamment les uns des autres, un alkyle à 1 ou 2 atomes de carbone, un benzyle ou un radical $H$-$(OC_2H_4)$-$_n$ dans lequel n est égal à 1 ou à 2,

et

$A_1$ et $Z_1$ ont les significations données à la revendication 3.

6. Procédé selon la revendication 5 caractérisé en ce que:

$A_1$ représente un phényle éventuellement porteur d'un radical alkyle ou d'un radical alcoxy qui contiennent chacun 1 ou 2 atomes de carbone, ou d'un chlore, le cycle phénylique pouvant porter un ou deux des substituants,

$Z_1$ représente un radical phénylène-1,4 éventuellement porteur d'un méthoxy, d'un hydroxy ou d'un chlore, le cycle phénylène pouvant porter un ou deux des substituants,

et

$B_1$, $R_1$, $R_2$ et $R_3$ ont les significations qui leur ont été données à la revendication 5.

7. Procédé selon la revendication 6 caractérisé en ce que:

$A_1$ représente un phényle éventuellement porteur d'un méthyle, d'un méthoxy ou d'un chlore, le cycle phénylique pouvant porter un ou deux des substituants,

$R_1$, $R_2$ et $R_3$ représentent chacun, indépendamment les uns des autres, un radical méthyle, benzyle ou $HO$-$C_2H_4$-,

et

$Z_1$ et $B_1$ ont les significations données à la revendication 6.